Europäisches Patentamt

European Patent Office

Office européen des brevets

(19)

(11) Veröffentlichungsnummer: **0 031 553**
**B1**

(12)

# EUROPÄISCHE PATENTSCHRIFT

(45) Veröffentlichungstag der Patentschrift:
14.12.83

(51) Int. Cl.³: **C 12 N 9/04**

(21) Anmeldenummer: **80108022.7**

(22) Anmeldetag: **18.12.80**

(54) Verfahren zur Gewinnung von Glycerin-dehydrogenase.

(30) Priorität: **27.12.79 DE 2952410**

(43) Veröffentlichungstag der Anmeldung:
**08.07.81 Patentblatt 81/27**

(45) Bekanntmachung des Hinweises auf die Patenterteilung:
**14.12.83 Patentblatt 83/50**

(84) Benannte Vertragsstaaten:
**AT BE CH DE FR GB IT LI LU NL SE**

(56) Entgegenhaltungen:
**GB - A - 2 021 594**

**APPLIED BIOCHEMISTRY & MICROBIOLOGY, Band 13, Nr. 5, Oktober 9, 1977, Plenum Publishing Corp. M.B. OVERCHENKO et al.: "Isolation of a preparation of glycerol dehydrogenase from Acetobacter suboxydans and its characterization", Seiten 509-513**
**CHEMICAL ABSTRACTS, Band 92, Nr. 1, 7. Januar 1980, Zusammenfassung 2194q, Seite 221 COLUMBUS OHIO (US) & J. Bacteriol. 1979, 140(1), 182-7 C.T. TANG et al.: "Purification and properties of a nicotinamide adenine dinucleotide-linked dehydrogenase that serves an Escherichia coli mutant for glycerol catabolism".**
**CHEMICAL ABSTRACTS, Band 91, Nr. 13, 24. September 1979, Zusammenfassung Nr. 106580h, Seite 472 COLUMBUS OHIO (US) & J. Ferment. Technol. 1979,**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Stahl, Peter, Dr., Jägerstrasse 12, D-8131 Bernried (DE)**
Erfinder: **Seidel, Hans, Dr., Waxensteinstrasse 6, D-8132 Tutzing (DE)**
Erfinder: **Brunner, Herwig, Dr., Paradeisstrasse 20 D, D-8120 Weilheim (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

(56) Entgegenhaltungen: (Fortsetzung)
**57(3), 221-6 YAMADA, SHIGEKI et al.: "Studies on aerobic fermentation. V. Enzymatic production of dihydroxyacetone by Acetobacter suboxydans ATCC 621".**
**CHEMICAL ABSTRACTS, Band 90, Nr. 17, 23. April 1979, Zusammenfassung 136250x, Seite 379 COLUMBUS OHIO (US)**
**CHEMICAL ABSTRACTS, Band 89, Nr. 23, 4. Dezember 1978, Zusammenfassung 193661k, Seite 302**

56  Entgegenhaltungen: (Fortsetzung)
COLUMBUS OHIO (US) & FEMS Microbiol. Lett. 1978, 4(4), 185-9 S. HUETING et al.: "Properties and regulation of synthesis of the glycerol dehydrogenase present in Klebsiella aerogenes NCTC 418, growing in chemostat culture".
CHEMICAL ABSTRACTS, Band 81, Nr. 23, 9. Dezember 1974, Zusammenfassung 150322t, Seite 396 COLUMBUS OHIO (US)
CHEMICAL ABSTRACTS, Band 62, Nr. 6, 15 März 1965, Spalte 6726a COLUMBUS OHIO (US) & Can. J. Biochem. 43(1), 122-5 (1965) JEAN HIMMS-HAGEN et al.: "Preparation of glycerol dehydrogenase for enzymic estimation of glycerol".
CHEMICAL ABSTRACTS, Band 54, Nr. 20, 25.10.1960, Spalte 21333b COLUMBUS OHIO (US) & J. Biol. Chem. 235, 1820-3 (1960) EDMUND C.C. LIN et al.: "Activation of glycerol dehydrogenase from Aerobacter aerogenes by univalent cations".

Verfahren zur Gewinnung von Glycerin-dehydrogenase

Die Erfindung betrifft ein Verfahren zur Gewinnung von Glycerin-dehydrogenase aus Mikroorganismen und eine neue Glycerin-dehydrogenase, die gegenüber bekannten Glycerin-dehydrogenasen eine wesentlich niedrigere $K_M$ aufweist.

Glycerin-dehydrogenase hat Bedeutung vor allem bei der Bestimmung von Glycerin. Letzteres wird bei der Triglycerid-Spaltung freigesetzt. Die Glycerinbestimmung mit Glycerin-dehydrogenase nach der Gleichung

Glycerin + NAD $\xrightarrow{\text{Glyc-DH}}$ Dihydroxyaceton + NADH, wobei NADH nach einer der hierfür bekannten Methoden gemessen wird, spielt daher eine wesentliche Rolle im Rahmen der Triglycerid-Bestimmung, bei welcher aus dem Triglycerid zuerst durch Lipase und gegebenenfalls Esterase eine äquivalente Menge Glycerin in Freiheit gesetzt wird.

Dieses Verfahren wird dadurch belastet, dass die Glycerin-dehydrogenase nach den bisher bekannten Verfahren zu ihrer Gewinnung in relativ niedriger Ausbeute anfällt und durch die erforderliche lange Fermentationsdauer bei der Züchtung der Mikroorganismen die Aufwendungen zu hoch liegen. So wurden mit den bekannten Verfahren nur Aktivitätsausbeuten von etwa 40 U./l Medium bei ca. 24 Stunden Kultivierungsdauer erreicht.

Der Erfindung liegt daher die Aufgabe zugrunde, ein Verfahren zu schaffen, mit welchem bei gleichem oder verringertem Aufwand wesentlich höhere Enzymmengen erhältlich sind.

Gelöst wird diese Aufgabe erfindungsgemäss durch ein Verfahren zur Gewinnung von Glycerin-dehydrogenase aus Mikroorganismen durch aerobe Züchtung eines Glycerin-dehydrogenase bildenden Mikroorganismus in einem geeigneten glycerinhaltigen Nährmedium und Gewinnung des Enzyms aus der Biomasse oder dem Nährmedium, welches dadurch gekennzeichnet ist, dass man den Mikroorganismus zuerst unter aeroben Bedingungen, danach unter anaeroben Bedingungen, d.h. unter $O_2$-Ausschluss, züchtet. Vorzugsweise züchtet man Aerobacter aerogenes DSM 1643 oder NCIB 418.

Es hat sich gezeigt, dass mit den erfindungsgemäss verwendeten Mikroorganismen ohne Änderung sonstiger Bedingungen bereits mindestens 10fach bessere Aktivitätsausbeuten erzielt werden können. Dabei werden mit Aerobacter aerogenes DSM 1643 verbesserte Ausbeuten auch dann erhalten, wenn man bekannte Gewinnungsverfahren anwendet, bei welchen also nur anaerob oder nur aerob gezüchtet wird.

Vorzugsweise hält man die aeroben Bedingungen, also die Zufuhr von Luft oder Sauerstoff bzw. sauerstoffhaltigen Gasen, bis zum Ende der log-Phase aufrecht. Unter log-Phase wird die exponentielle oder logarithmische Wachstumsphase verstanden, die durch eine konstante maximale Teilungsrate charakterisiert ist.

Es ist bekannt, dass Glycerin-dehydrogenase bildende Mikroorganismen zumeist dieses Enzym dann am besten bilden, wenn sie unter anaeroben Bedingungen, also unter Sauerstoffausschluss, gezüchtet werden. In Anwesenheit von Sauerstoff tritt in den meisten Fällen eine Verschiebung der Stoffwechselleistungen auf und anstelle von Glycerin-dehydrogenase wird Glycerin-3-phosphat-dehydrogenase gebildet und für den Glycerinabbau nach vorheriger Phosphorylierung mit Glycerinkinase verwendet. Jedoch wurden auch schon Mikroorganismen beschrieben, welche Glycerin-dehydrogenase bei Belüftung bilden (JA-OS 0 040 737). Demgegenüber wurde nunmehr gefunden, dass bei der oben beschriebenen Aufeinanderfolge aerober und anaerober Bedingungen wesentlich höhere Ausbeuten an Enzym erzielbar sind als bei einer reinen aeroben oder reinen anaeroben Kultivierung. Besonders ausgeprägt ist dies bei den beiden oben aufgeführten Mikroorganismen der Fall.

Im übrigen erfolgt die Züchtung der Mikroorganismen und die Gewinnung des Enzyms aus den geernteten Mikroorganismen nach den hierfür bekannten Methoden. So enthält ein bekanntes, im Rahmen der Erfindung verwendbares Medium Glycerin, Pepton, Fleischextrakt, Hefeextrakt und NaCl sowie Puffersubstanz. Glycerin wird dabei als einzige Kohlenstoffquelle in relativ grosser Menge von etwa 40 g/l eingesetzt und im Laufe der Wachstumsdauer, die bei dem bekannten Verfahren etwa 24 Stunden beträgt, verbraucht. Im Rahmen der vorliegenden Erfindung wurde jedoch gefunden, dass noch bessere Ergebnisse erzielt werden, wenn man bei der aeroben/anaeroben Züchtungsmethode in der anaeroben Phase den Glyceringehalt wieder steigert, d.h. mehr Glycerin zusetzt, als durch den Mikroorganismus verbraucht wird. Vorzugsweise wird daher der Glyceringehalt so eingestellt, dass er bis zum Ende der logarithmischen Wachstumsphase auf etwa 0,3 bis 0,5% abgesunken ist, und wird dann während der anaeroben Phase wieder auf 0,8 bis 1,5% gesteigert, also etwa auf den Ausgangswert.

Ebenso erwies es sich als vorteilhaft, anstelle von Hefeextrakt Biotin dem Medium zuzusetzen. Die bevorzugte Biotinkonzentration beträgt 10 bis 100 µg/l.

Die Züchtungstemperatur liegt im üblichen Rahmen, für die angestrebten günstigen Ergebnisse sollte die Temperatur zwischen 25 und 40 °C gehalten werden.

Der pH-Wert liegt zweckmässig zwischen 6 und 9, wobei zur pH-Werteinstellung übliche Puffersubstanzen verwendet werden. Besonders gute Ergebnisse lieferte ein Phosphationen und Ammoniumionen enthaltender Puffer.

Das erfindungsgemässe Verfahren läuft wesentlich rascher ab als die bekannten Verfahren. In der Regel werden bereits nach etwa 6- bis 7stündiger Fermentation optimale Enzymaktivitätswerte erzielt. Das Ende der Züchtung lässt sich leicht durch ein beginnendes Abfallen des pH-Werts erkennen.

Bei Anwendung der bevorzugten Bedingungen gelingt es, Enzymaktivitätsausbeuten von 9000 U./l und darüber erfindungsgemäss zu erzielen. Dies entspricht einer mehr als 100fachen Steigerung gegenüber dem bekannten Verfahren.

Eine vorteilhafte Besonderheit des erfindungsgemässen Verfahrens liegt weiter darin, dass bei Züchtung von Aerobacter aerogenes DSM 1643 eine Glycerin-dehydrogenase erhalten wird, die sich von bekannten Glycerin-dehydrogenasen durch eine wesentlich niedrigere Michaelis-Konstante $K_M$ auszeichnet. Während sie bei bekannten Enzymen relativ gross ist und im Bereich von 1 bis $4 \times 10^{-2}$ liegt, weist das Enzym aus dem vorgenannten erfindungsgemäss verwendeten Stamm eine $K_M$ von $6,6 \times 10^{-4}$ auf, liegt also zwei Zehnerpotenzen niedriger. Aufgrund dieser vorteilhaften Eigenschaften wird unter sonst gleichen Bedingungen bei Verwendung dieses Enzyms eine bestimmte Umsatzrate mit deutlich geringeren Enzymmengen erzielt als bei bekannten Glycerin-dehydrogenasen. Daher ist eine Glycerin-dehydrogenase mit einer $K_M$ von $6,6 \times 10^{-4}$ M ein weiterer Gegenstand der Erfindung.

Wie bereits erwähnt, kann das gewünschte Enzym aus der Biomasse der Mikroorganismen nach üblichen Methoden gewonnen werden. Beispielsweise kann die Kultursuspension direkt durch Ultraschall aufgeschlossen und nach Abtrennung unlöslicher Bestandteile das Enzym aus dem Rohextrakt mit üblichen Fällungsmitteln, wie z.B. Ammonsulfat, isoliert werden. Bei Verwendung von Ammonsulfat wird zweckmässig bei 35 bis 45% Sättigung fraktioniert. Eine weitere Anreicherung kann beispielsweise durch einen Erhitzungsschritt erfolgen, zweckmässig 2 bis 10 Minuten bei 50 bis 70°C. Hierbei wird ein Teil der Verunreinigungen denaturiert und kann abfiltriert oder abzentrifugiert werden. Man erhält so ein Enzym mit einer spezifischen Aktivität von etwa 20 U./mg.

Die Züchtung erfolgt wie üblich in der Schüttelkultur oder unter Rühren.

Die folgenden Beispiele erläutern die Erfindung weiter.

Beispiel 1

Aerobacter aerogenes DSM 1643 wird in einem Medium, welches 0,8% Caseinpepton, 0,8% Fleischpepton, 0,3% $K_2HPO_4$, 0,2% $NH_4(2.HPO_4)$, 0,6% NaCl, 1 bis 2% Glycerin und 50 µg Biotin enthält, bei 37°C und einem pH-Wert von 7,0 unter Belüftung und Rühren in einem 10-l-Fermenter gezüchtet. Pro Stunde werden 300 l Luft zugeführt, das Rühren erfolgt mit 300 Upm bis zum Ende der logarithmischen Wachstumsphase.

Nach Ende der logarithmischen Wachstumsphase wird die Luftzufuhr beendet und mit der Zugabe von Glycerin begonnen. Die Glycerin-Konzentration steigt dabei von 0,3 bis 0,5% auf 1,0% bis zum Ende der Fermentation (etwa 6 Stunden). Man erhält so eine Biomasse von 7 bis 10 g/l und eine Aktivität von 7000 bis 9000 U./l.

Die erhaltene Kultursuspension wird im Ultraschallgerät 3 Minuten aufgeschlossen. Unlösliche Bestandteile werden abzentrifugiert, und im klaren Überstand erfolgt die Aktivitätsbestimmung wie folgt:

Testlösungen:
1. Puffer-Ammonsulfatlösung
   $NaHCO_3$ (MG = 84,01): 0,12 M = 9,69 g/l
   $(NH_4)_2SO_4$ (MG = 132,15): 0,04 M = 5,08 g/l
   mit 1n NaOH auf pH 10,0.
2. NAD-Lösung (MG = 663,4)
   c = 10–100: je nach verwendeter Rohextraktprobe.
3. 1,5 M Glycerin
   12,9 ml 87% Glycerin auf 100 ml bidest. Wasser
4. Physiologische Kochsalzlösung (0,85% NaCl)
   0,1 M $PO_4$-Puffer, pH 7.

Testansatz:

| | |
|---|---|
| Massstrahl: | 366 nm |
| Testvolumen: | 3,05 ml |
| Schichtdicke: | 1 cm |
| Temperatur: | 25°C |

| | | |
|---|---|---|
| Puffer/$(NH_4)_2SO_4$ | (1) | 2,65 ml |
| NAD | (2) | 0,10 ml |
| Probe | | 0,10 ml |
| Mischen, Verlauf abwarten | | |
| Start: Glycerin | (3) | 0,20 ml |

Berechnung:
$$\frac{\Delta E \cdot 3,05 \cdot 1000}{3,4 \cdot 0,1 \cdot 1} = U./l$$

Zur weiteren Reinigung des Enzyms wird der Rohextrakt mit Ammonsulfatlösung fraktioniert, wobei die Fraktion 35 bis 45% Sättigung gewonnen und in 0,1 M Phosphat-Puffer, pH 7, enthaltend 0,85% NaCl, aufgelöst wird. Die Lösung wird 4 Minuten bei 60°C erhitzt und zentrifugiert. Man erhält eine klare Glycerin-dehydrogenase-Lösung mit einer spezifischen Aktivität von etwa 20 U./mg.

Die Bestimmung der $K_M$ an diesem Präparat ergab den Wert $6,6 \times 10^{-4}$ M.

Beispiel 2

Das Verfahren von Beispiel 1 wurde wiederholt unter Verwendung von Aerobacter aerogenes NCIB 418 (auch als Enterobacter aerogenes bezeichnet). Die Züchtungsdauer betrug 8 Stunden, die Aktivität im Rohextrakt 3000 bis 4000 U./l.

**Patentansprüche**

1. Verfahren zur Gewinnung von Glycerin-dehydrogenase aus Mikroorganismen durch aerobe Züchtung eines Glycerin-dehydrogenase bildenden Mikroorganismus in einem geeigneten glycerinhaltigen Nährmedium und Gewinnung des Enzyms aus der Biomasse oder dem Nährmedium, dadurch gekennzeichnet, dass man den Mikroorganismus zuerst unter aeroben Bedingungen und danach unter anaeroben Bedingungen züchtet.

2. Verfahren nach Anspruch 1, dadurch gekennzeichnet, dass man Aerobacter aerogenes DSM 1643 oder NCIB 418 züchtet.

3. Verfahren nach Anspruch 2, dadurch gekennzeichnet, dass man die aeroben Bedingungen bis zum Ende der log-Phase aufrechterhält.

4. Verfahren nach Anspruch 2 oder 3, dadurch gekennzeichnet, dass man in der anaeroben Züchtungsphase dem Nährmedium mehr Glycerin zusetzt, als durch den Mikroorganismus verbraucht wird.

5. Verfahren nach Anspruch 4, dadurch gekennzeichnet, dass man in der anaeroben Phase den Glyceringehalt des Nährmediums von 0,3 bis 0,5% auf 0,8 bis 1,5% steigert.

6. Verfahren nach einem der vorhergehenden Ansprüche, dadurch gekennzeichnet, dass man dem Nährmedium Biotin zusetzt.

7. Verfahren nach einem der vorhergehenden Ansprüche, dadurch gekennzeichnet, dass man bei einer Temperatur zwischen 25 und 40 °C züchtet.

8. Glycerin-dehydrogenase aus Mikroorganismen, gekennzeichnet durch eine $K_M$ von $6,6 \times 10^{-4}$ M.

9. Verfahren zur Gewinnung von Glycerin-dehydrogenase aus Mikroorganismen durch aerobe Züchtung eines Glycerin-dehydrogenase bildenden Mikroorganismus in einem geeigneten glycerinhaltigen Nährmedium und Gewinnung des Enzyms aus der Biomasse oder dem Nährmedium, dadurch gekennzeichnet, dass man Aerobacter aerogenes DSM 1643 züchtet.

## Claims

1. Process for obtaining glycerol dehydrogenase from micro-organisms by aerobic culturing a glycerol dehydrogenase-forming micro-organism in an appropriate glycerol-containing nutrient medium and obtaining of the enzyme from the biomass or the nutrient medium, characterised in that one first cultures the micro-organism under aerobic conditions and thereafter under anaerobic conditions.

2. Process according to claim 1, characterised in that one cultures Aerobacter aerogenes DSM 1643 or NCIB 418.

3. Process according to claim 2, characterised in that one maintains the aerobic conditions up to the end of the log phase.

4. Process according to claim 2 or 3, characterised in that, in the anaerobic culture phase, one adds more glycerol to the nutrient medium than is used up by the micro-organism.

5. Process according to claim 4, characterised in that, in the anaerobic phase, one increases the glycerol content of the nutrient medium from 0.3 to 0.5% up to 0.8 to 1.5%.

6. Process according to one of the preceding claims, characterised in that one adds biotin to the nutrient medium.

7. Process according to one of the preceding claims, characterised in that one cultures at a temperature between 25 and 40 °C.

8. Glycerol dehydrogenase from micro-organisms, characterised by a $K_M$ of $6.6 \times 10^{-4}$ M.

9. Process for obtaining glycerol dehydrogenase from micro-organisms by the aerobic culturing of a glycerol dehydrogenase-forming micro-organism in an appropriate glycerol-containing nutrient medium and obtaining the enzyme from the biomass or the nutrient medium, characterised in that one cultures Aerobacter aerogenes DSM 1643.

## Revendications

1. Procédé d'obtention de la glycérol-déshydrogénase à partir de microorganismes par culture aérobie d'un microorganisme formant de la glycérol-déshydrogénase dans un milieu nutritif contenant du glycérol approprié et obtention de l'enzyme à partir de la biomasse ou du milieu nutritif, caractérisé en ce qu'on cultive d'abord le microorganisme dans des conditions aérobies puis dans des conditions anaérobies.

2. Procédé suivant la revendication 1, caractérisé en ce qu'on cultive Aerobacter aerogenes DSM 1643 ou NCIB 418.

3. Procédé suivant la revendication 2, caractérisé en ce qu'on maintient les conditions aérobies jusqu'à la fin de la phase log.

4. Procédé suivant la revendication 2 ou 3, caractérisé en ce qu'on ajoute au milieu nutritif dans la phase de croissance anaérobie davantage de glycérol que n'en consomme le microorganisme.

5. Procédé suivant la revendication 4, caractérisé en ce que dans la phase anaérobie, on élève la teneur en glycérol du milieu nutritif de 0,3 à 0,5% jusqu'à 0,8 à 1,5%.

6. Procédé suivant l'une des revendications précédentes, caractérisé en ce qu'on ajoute de la biotine au milieu nutritif.

7. Procédé suivant l'une des revendications précédentes, caractérisé en ce qu'on cultive à une température entre 25 et 40 °C.

8. Glycérol-déshydrogénase provenant de microorganismes, caractérisée par une $K_M$ de $6,6 \times 10^{-4}$ M.

9. Procédé d'obtention de glycérol-déshydrogénase à partir de microorganismes par culture aérobie d'un microorganisme formant de la glycérol-déshydrogénase dans un milieu nutritif glycériné approprié et obtention de l'enzyme à partir de la biomasse ou du milieu nutritif, caractérisé en ce qu'on cultive Aerobacter aerogenes DSM 1643.